# EUROPÄISCHE PATENTSCHRIFT

(11) **EP 2 442 679 B1**
(45) Veröffentlichungstag und Bekanntmachung des Hinweises auf die Patenterteilung: **19.10.2022**
(21) Anmeldenummer: 10721341.5
(22) Anmeldetag: 22.05.2010
(51) Int. Cl.: A23L 3/26, G01N 21/03, C12M 1/34, G01N 21/80, G01N 21/77, A61L 2/08, A61L 2/28

(54) **SENSORVORRICHTUNG UMFASSEND EINEN OPTISCHEN SENSOR, EINEN BEHÄLTER UND EIN KOMPARTIMENTIERUNGSMITTEL**
SENSOR DEVICE COMPRISING AN OPTICAL SENSOR, A CONTAINER AND A COMPARTMENTALIZATION MEANS
DISPOSITIF DÉTECTEUR COMPRENANT UN CAPTEUR OPTIQUE, UN RECIPIENT ET UN MOYEN DE COMPARTIMENTATION

(30) Priorität: 19.06.2009 DE 102009025520; 23.10.2009 DE 102009050448
(43) Veröffentlichungstag der Anmeldung: 25.04.2012
(73) Patentinhaber: Sartorius Stedim Biotech GmbH, 37079 Göttingen (DE)
(72) Erfinder: BAUMFALK, Reinhard, 37075 Göttingen (DE); WEISSHAAR, Stefan, 37139 Adelebsen (DE); GRELLER, Gerhard, 37085 Göttingen (DE); RIECHERS, Daniel, 93051Regensburg (DE); LÜDERS, Julia, 31246 Lahstedt (DE); POLLOSEK, Peter, 37194 Bodenfelde OT Amelieth (DE)
(74) Vertreter: Novagraaf International SA
(86) Internationale Anmeldenummer: PCT/EP2010/003158
(87) Internationale Veröffentlichungsnummer: WO 2010/145747

(56) Entgegenhaltungen:
- EP-A2- 0 351 516
- EP-A2- 2 065 701
- WO-A1-2010/017519
- WO-A2-2005/118771
- DE-U1-202007 005 399
- GOPALA RAO G AND NARAYANA RAO V: "Ascorbic acid as a reducing agent in quantitative analysis" Originalabhandlungen FRESENIUS' JOURNAL OF ANALYTICAL CHEMISTRY Bd. 147, Nr. 5, 1. September 1955 (1955-09-01), Seiten 338-347, XP002595390 Gefunden im Internet: URL:http://www.springerlink.com/content/l2 3u3112m475w109 [gefunden am 2010-08-05]

## Beschreibung

Die Erfindung betrifft eine Sensorvorrichtung, aufweisend mindestens einen optischen Sensor, einen Behälter und ein Kompartimentierungsmittel. Optische Sensoren werden insbesondere in Einweg-, Misch- und Bioreaktoren bzw. -behältern in der Medizintechnik und Biotechnologie eingesetzt. In diesen und ähnlichen Anwendungsgebieten ist es oft nötig, einen Behälter vor der Benutzung zu sterilisieren. Auf dem Gebiet der Einwegprodukte hat sich eine Sterilisation mittels Strahlung, insbesondere GammaStrahlung, bewährt, welche jedoch schädlich für optische Sensoren ist. Dies betrifft insbesondere auf porösen Matrizes basierende optische Sensoren, wie beispielsweise fluoreszenzbasierte pH-Sensoren. Daher wird für derartige Sensoren ein effektives Schutzsystem benötigt, das zugleich kostengünstig realisiert werden kann.

Aus WO 02/056023 A1 und DE 10,051,220 A1 sind optische Sensoren zur Messung mindestens eines Parameters in einer Probe bekannt. Diese Sensoren basieren auf einer Vorrichtung zur Anregung der Fluoreszenz eines in einem Probengefäß bzw. Reaktor in einer Matrix immobilisierten Analyt-sensitiven Fluoreszenzfarbstoffes, der in zumindest indirektem Kontakt zur Probe steht, und einer Auswertungsvorrichtung für das resultierende Fluoreszenzantwortsignal. Die Auswertung bzw. die Bestimmung der Analyt-Konzentration kann hierbei sowohl durch die Verwertung der Fluoreszenzabklingzeit als auch der Fluoreszenzintensität erfolgen. Nachteilig ist, dass derartige pH-Sensorpatches, basierend auf einer hydrophilen Trägermatrix, wie z.B. imprägnierten Papieren oder Sol-Gel-Matrizes, bei einer Strahlen-Sterilisation dosisabhängig geschädigt werden. Es verringern sich sowohl die Intensität der Fluoreszenz des Farbstoffs bzw. der Farbstoffe, als auch die Sensitivität des Sensorpatches gegenüber der Messgröße.

Aus US 7,390,462 B2 ist ein Sensor bekannt, bei dem der Fluoreszenzfarbstoff in einer hydrophilen Matrix immobilisiert vorliegt. Hierbei wird ein Sensor mit dem pH-sensitiven Fluoreszenzfarbstoff MA-HPDS in einem Hydrogel vorliegend beansprucht. Auch hierbei ist nachteilig, dass derartige hydrophile optische Sensoren bei einer Sterilisation mit Gammastrahlung dosisabhängig geschädigt werden. Eine solche Strahlung wird insbesondere in der Labortechnologie bei Behältern aus Polymeren angewendet. Es verringern sich sowohl die Intensität der Fluoreszenz des Farbstoffs bzw. der Farbstoffe, als auch die Sensitivität des Sensors gegenüber der Messgröße. Eine besonders starke Schädigung eines solchen Sensorpatches tritt auf, wenn er während der Strahlen-Sterilisation in Kontakt mit einem größeren Volumen an Luft, bzw. auch mit üblichen Schutzgasen wie z.B. Stickstoff oder Argon steht. Bei der Strahlen-Sterilisation werden die Gase teilweise ionisiert, bzw. es entstehen freie Radikale. Diese Radikale reagieren beispielsweise bei der Sterilisation eines gasgefüllten Polymer-Beutels an den Wänden oder auch an den Sensoroberflächen ab. Auf porösen, hydrophilen Matrizes basierende Sensoren sind hierfür besonders anfällig, da die Sensorchemie prinzipbedingt an der Oberfläche, bzw. inneren Oberfläche der Matrix immobilisiert vorliegen muss, damit die zu vermessende Probe mit der Sensorchemie in Kontakt kommen kann. Das Ausmaß der Schädigung hängt einerseits von der Bestrahlungsdosis und andererseits vom Verhältnis der Oberfläche zum Volumen des bestrahlten, den Sensorpatch enthaltenden, Behälters ab. Dieses Verhältnis bestimmt die Anzahl an Ionen bzw. Radikalen welche den Sensorpatch, bzw. die darin enthaltene Sensorchemie schädigen.

Aus DE 10 2009 003 971.6 A1 ist ein optischer Sensor zur Messung mindestens eines Parameters bekannt, welcher porös mit einer Edelmetallschicht überzogen ist, womit eine Abreaktion von reaktiven Teilchen an der Edelmetallschicht erreicht wird. Hierbei ist jedoch nachteilig, dass sich eine derartige Beschichtung technisch und mechanisch aufwendig gestaltet und mit hohen Kosten verbunden ist, was insbesondere im Bereich von Einwegprodukten zu vermeiden ist.

Die DE 20 2007 005399 U1 offenbart einen Einwegbioreaktor mit reversibel, äußerlich anbringbarer Sensoranordnung zur Messung einer physikalischen Größe eines enthaltenen Mediums. Hierbei wird durch eine Peripherieleitung am Zu- und/oder Abfluss eine Sensoranordnung außerhalb des Einwegbioreaktors angebracht. Dies ist technisch aufwendig und mit hohen Kosten verbunden.

Aus EP 2 065 701 A2 ist eine Messanordnung bekannt, mit einem flexiblen Einwegbehälter und einer Einwegmesssonde zur Messung einer analytischen Eigenschaft eines Mediums. Durch eine Kapsel wird ein Schutzbereich für die Messsonde geschaffen, wobei die Kapsel mit einem Reduktionsmittel gefüllt ist. Optische Sensoren werden hierbei nicht erörtert.

Die WO 2005/118771 A2 offenbart ein Einwegbioreaktorsystem. Die Thematik der Schädigung eines optischen Sensors, welcher im Bioreaktorinnenraum angeordnet ist und durch Gammabestrahlung ausgelöst wird, ist nicht erörtert.

Die WO 2010/017519 A1 stellt für die vorliegende Erfindung Stand der Technik nach Artikel 54(3) EPÜ dar und offenbart eine Sensorvorrichtung, welche nach der Sterilisation mit einem Einwegbehälter mittels Sterilkonnektoren verbunden wird.

Der vorliegenden Erfindung liegt daher die Aufgabe zugrunde, eine Sensorvorrichtung für einen optischen Sensor zu schaffen, womit die Empfindlichkeit des optischen Sensors gegenüber Strahlung, insbesondere Gammastrahlung, herabgesetzt ist, und welche kostengünstig und einfach realisierbar ist.

Die Aufgabe wird durch eine Sensorvorrichtung gemäß Anspruch 1 gelöst. Die erfindungsgemäße Sensorvorrichtung weist mindestens einen optischen Sensor, einen Behälter und ein Kompartimentierungsmittel auf. Das Kompartimentierungsmittel teilt den Behälter temporär in mindestens zwei Räume, wobei in dem dadurch entstandenen Nebenraum der optische Sensor enthalten ist. Auch die Unterbringung von zwei oder mehr Sensoren in dem Nebenraum ist ausführbar. Die Kompartimentierung kann erfindungsgemäß, beispielsweise nach einer Sterilisation oder einem Transportvorgang, rückgängig gemacht werden.

Der Ausdruck Kompartimentierung, wie hierin verwendet, unterliegt keiner Einschränkung und betrifft jeden Vorgang, der geeignet ist einen Raum in mindestens zwei Räume aufzuteilen. Analog ist der Ausdruck Kompartimentierungsmittel zu betrachten.

Gemäß einer bevorzugten Ausführungsform der Erfindung ist der optische Sensor durch Strahlung unter Erhalt seiner Funktionsfähigkeit sterilisierbar. Die Empfindlichkeit gegenüber einer Sterilisierung, beispielsweise mittels ionisierender Strahlung, Gammastrahlung, UV-C-, Beta- oder Elektronenstrahlung ist durch das Kompartimentierungsmittel herabgesetzt. Durch die Verringerung des Volumens an Gas in umgebender Nähe zum optischen Sensor, verringert sich entsprechend die Anzahl daraus gebildeter reaktiver Teilchen, welche Zugang zu dem optischen Sensor haben und mit dessen Matrix und dem Fluoreszenzfarbstoff bzw. den Fluoreszenzfarbstoffen reagieren.

Somit wird ein besseres Signal-Rausch-Verhältnis, sowie eine allgemein höhere Sensitivität des optischen Sensors gegenüber seiner Messgröße erreicht. Die Messgrößen können hierbei z.B. der pH-Wert, die Gelöstsauerstoffkonzentration oder andere Parameter sein. Die Kompartimentierung bzw. das Kompartimentierungsmittel ist nach der Strahlensterilisation bzw. vor der Verwendung des Behälters, beispielsweise als Bioreaktor, erfindungsgemäß rückgängig machbar, ohne die Sterilbarriere des Behälters zu brechen.

Gemäß einer weiteren bevorzugten Ausführungsform der Erfindung ist durch das Kompartimentierungsmittel der Nebenraum von dem Hauptraum gasdicht abgrenzbar. Somit wird jegliche Diffusion reaktiver Teilchen verhindert. Eine Evakuierungsvorrichtung, welche vorzugsweise in Form einer thermoplastisch verschließbaren Leitung oder über ein Ventil regelbar ist, führt zusätzlich zu einer Gasverringerung, womit die Anzahl entstehender reaktiver Teilchen bei einer Strahlensterilisation auf ein Minimum reduziert ist.

In einer weiteren bevorzugten Ausführungsform der Erfindung verschließt das Kompartimentierungsmittel den Nebenraum gegenüber dem Hauptraum partiell. Eine freie Diffusion der reaktiven, durch eine Strahlensterilisation entstehenden Teilchen ist nur im lichten Gasraum zu erwarten, so dass kleine Spalte welche die beiden Kompartimente verbinden, aufgrund der Desaktivierung der reaktiven Teilchen bei Stößen mit den Wänden, zulässig sind. Zudem ist eine derartige Ausführungsform herstellungstechnisch einfach und kostengünstig realisierbar. Eine Evakuierungsvorrichtung kann hierbei dem gesamten Behälter Gas entziehen und somit weiterhin die Quantität sich bildender reaktiver Teilchen reduzieren.

Gemäß einer besonders bevorzugten Ausführungsform der Erfindung ist das Kompartimentierungsmittel als eine den optischen Sensor bedeckende Kappe ausgebildet. Die Kappe kann passgenau über den Sensor oder eine entsprechende Vorrichtung gestülpt werden und zusätzlich eine dauerhafte Verbindung beispielsweise an den Sensorrahmen oder den Behälter besitzen um kein Störobjekt darzustellen.

In einer weiteren vorteilhaften Ausgestaltung der Erfindung ist das Kompartimentierungsmittel ein den optischen Sensor enthaltender Schlauch. Dieser kann beispielsweise von der Behälterinnenwand abstehend über den optischen Sensor gesteckt werden und somit eine Kompartimentierung schaffen. Ein Pfropfen oder entsprechende herstellungstechnische Maßnahmen können zusätzlich das Innenvolumen des Schlauches reduzieren und damit die Anzahl sich in der Umgebung des optischen Sensors bildender reaktiver Teilchen minimieren. Durch ein insofern verringertes Nebenraumvolumen wird ebenfalls der Totraum reduziert, welcher nach dem Abkapseln von dem optischen Sensor besteht.

Nach einer weiteren besonders bevorzugten Ausführungsform der Erfindung ist die Kompartimentierung durch die Wandung des Behälters ausgeführt. Dies kann beispielsweise durch eine Klammer, welche die Wandung zusammendrückt oder durch einen Druckverschluss, wie beispielsweise einen ZipLoc^{®}-Verschluss erreicht werden. Eine weitere Möglichkeit besteht darin, ein leicht durchtrennbares Material an die Stelle der Kompartimentierung zu schweißen oder zu kleben, bzw. den Behälter bei flexibler Ausgestaltung mit sich selbst zu verschweißen oder zu verkleben. Derartige Ausführungen ermöglichen ein hohes Einsparpotential und gewähren zusätzlich einen sicheren Schutz vor einer zu großen Menge an reaktiven Teilchen.

Gemäß einer weiteren bevorzugten Ausführungsform der vorliegenden Erfindung ist der Nebenraum ein den optischen Sensor aufnehmendes Port-System. Dieses kann eine oder mehrere Halteeinrichtungen besitzen, welche das Port-System fixieren. Um zusätzlich eine höhere Dichtigkeit zu erreichen weist das Portsystem mindestens eine Dichtung, beispielsweise in Form von O-Ringen, auf. Eine Evakuierungsvorrichtung, welche vorzugsweise in Form einer thermoplastisch verschließbaren Leitung oder über ein Ventil regelbar ist, kann zusätzlich zu einer Gasverringerung führen, womit die Anzahl entstehender reaktiver Teilchen bei einer Strahlensterilisation auf ein Minimum reduziert ist. Vorstehend genannte Ausführungsform erlaubt die Strahlen-Sterilisation des optischen Sensorelements unter vermindertem Gasdruck (< 1,01325 bar) durchzuführen.

Gemäß einem Beispiel der Erfindung bestimmt der optische Sensor mindestens einen Parameter in einem Medium. Der optische Sensor weist mindestens eine Matrix, die wenigstens einen Fluoreszenzfarbstoff enthält, welche von einem transparenten Träger getragen ist, auf.

Gemäß einem Beispiel der Erfindung ist die Matrix des optischen Sensors hydrophil. Besondere Ausführungen können eine Sol-Gel-Matrix oder ein Hydrogel sein, in welchem der Fluoreszenzfarbstoff immobilisiert vorliegt. Die hydrophile Eigenschaft ist besonders für optische Sensoren nötig, deren in der Matrix eingebettete Fluoreszenzfarbstoff für ein wässriges Medium zugänglich sein muss.

Optische Sensoren, welche eine hydrophile Matrix aufweisen sind naturgemäß äußerst sensibel gegenüber einer Sterilisierung, z.B. mittels Gammastrahlung. Eine Kompartimentierung schützt wirkungsvoll gegen die Einflüsse von durch Strahlung freigesetzten reaktiven Teilchen.

Gemäß einem weiteren Beispiel der Erfindung ist die Matrix des optischen Sensors porös.

In einer weiteren vorteilhaften Ausgestaltung der Erfindung ist der Behälter aus Kunststoff mit zumindest teilweiser flexibler Wandung. Insbesondere derartige Behälter, welche überwiegend zum Einmalgebrauch benutzt werden, benötigen kostengünstige und einfach herstellbare Komponenten, wie sie die vorliegende Erfindung darstellt.

Gemäß einer besonders bevorzugten Ausführungsform der vorliegenden Erfindung kann der Nebenraum zumindest teilweise mit einem wässrigen Reduktionsmittel befüllt werden. Dies ergibt zusätzlich zu dem Schutz des optischen Sensors durch den Nebenraum gegenüber reaktiver Teilchen auch einen Schutz vor einer direkten Radiolyse der Sensorchemie. Bei der Radiolyse, beispielsweise der Indikatormoleküle eines auf einer porösen Matrix basierenden optischen Sensors, werden durch die ionisierende Strahlung Radikal-Kationen erzeugt, welche durch radikalische Kettenreaktionen weitere Schäden an der Sensorchemie hervorrufen können. Überraschenderweise kann dieser schädigende und die Sensorperformance negativ beeinflussende Effekt durch eine Imprägnierung der Sensormatrix mit einem wässrigen Reduktionsmittel, wie beispielsweise 0,1 - bis dreiunddreißigprozentiger L-Ascorbinsäurelösung, größtenteils unterbunden werden. L-Ascorbinsäurelösung unterbindet als Radikalquencher wirksam radikalische Kettenreaktionen. Weiterhin geeignete Reduktionsmittel sind die Salze der L-Ascorbinsäure, Iso-Ascorbinsäure, Verbindungen aus der Klasse der Hydrochinone oder Glutathion.

Die Figuren 1 bis 5 zeigen unterschiedliche Ausführungsformen der Sensorvorrichtung.

In den Zeichnungen zeigen:
- Figur 1: einen schematischen Querschnitt durch ein Ausführungsbeispiel der Sensorvorrichtung, wobei ein Schlauch mit Pfropfen den optischen Sensor gegenüber dem Hauptraum kompartimentiert,
- Figur 2: einen schematischen Querschnitt durch ein Ausführungsbeispiel der Sensorvorrichtung, wobei eine Klammer als Kompartimentierungsmittel den Behälter in einen Nebenraum und einen Hauptraum kompartimentiert,
- Figur 3: einen schematischen Querschnitt durch ein Ausführungsbeispiel der Sensorvorrichtung, wobei ein Druckverschluss, im Vergrößerungsausschnitt im Querschnitt dargestellt, den Behälter in einen Nebenraum und einen Hauptraum kompartimentiert,
- Figur 4a: eine Schnittansicht durch ein Ausführungsbeispiel der Sensorvorrichtung, mit einem den optischen Sensor enthaltenden Port-System in kompartimentiertem Zustand,
- Figur 4b: eine Schnittansicht durch ein Ausführungsbeispiel der Sensorvorrichtung, mit einem den optischen Sensor enthaltenden Port-System in geöffnetem Zustand und
- Figur 5: eine Schnittansicht durch ein Ausführungsbeispiel eines Teils der Sensorvorrichtung mit Schutzkappe, ein Reduktionsmittel enthaltend,
- Figur 6: eine Schnittansicht durch ein Ausführungsbeispiel eines Teils der Sensorvorrichtung mit Schubsystem im geschlossenen Zustand,
- Figur 7: eine Schnittansicht durch ein Ausführungsbeispiel eines Teils der Sensorvorrichtung mit Schubsystem im geöffneten Zustand und
- Figur 8: ein Diagramm der Empfindlichkeit der Sensoren (pH/Phase°).

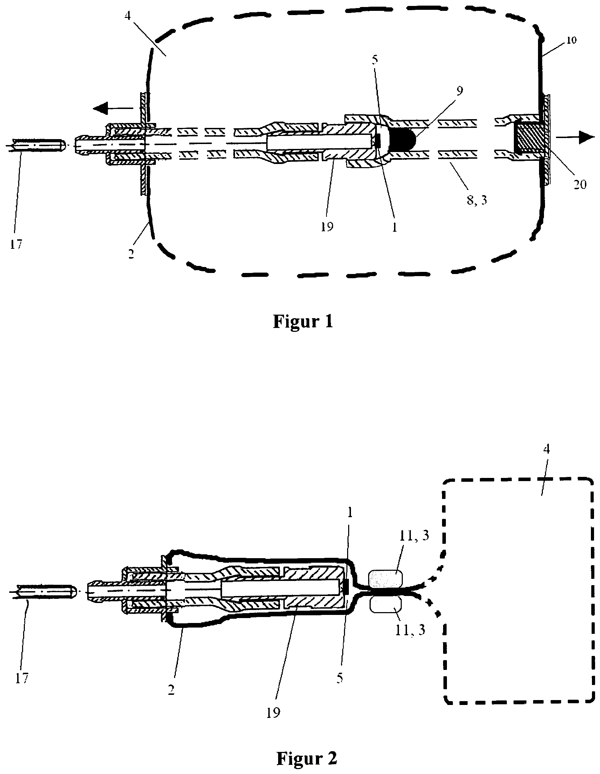

Gemäß Figur 1 bis Figur 4b besteht die Sensorvorrichtung aus einem optischen Sensor 1, einen Behälter **2** und ein Kompartimentierungsmittel **3.** Das Kompartimentierungsmittel **3** begrenzt den Behälter **2** temporär in einen Hauptraum **4** und einen Nebenraum **5**, welcher den optischen Sensor **1** enthält. Ein Lichtwellenleiter **17** ermöglicht die optische Messung eines oder mehrerer Parameter. Die Erreichung eines möglichst kleinvolumigen Nebenraumes ist ersichtlich.

Gemäß den Figuren 1 bis 3 ist der optische Sensor **1** auf einer lichtdurchlässigen Kappe **19**, beispielsweise aus Polycarbonat, aufgebracht.

Figur 1 zeigt einen schematischen Querschnitt durch ein Ausführungsbeispiel der Sensorvorrichtung, wobei ein Schlauch **8** oder Rohr mit Pfropfen **9** den optischen Sensor **1** gegenüber dem Hauptraum **4** kompartimentiert. Der Schlauch **8** kann beispielsweise aus Silikon gefertigt sein. An Stelle eines Pfropfens **9**, welcher nachträglich in den Schlauch **8** steckbar ist, kann auch ein Schlauch **8** verwendet werden, der bereits ein verringertes Gasvolumen besitzt. Der Schlauch **8** kann, wie hier dargestellt, auf einem Flansch **20** sitzen oder fest mit dem Behälter **2** verschweißt oder verklebt sein. Durch Zug an diesem und/oder an der Ansatzstelle des optischen Sensors **1** ist der Schlauch **8** von dem optischen Sensor **1** nach der Sterilisation trennbar, was in Pfeilrichtung dargestellt ist. In umgekehrter Weise ist vor der Sterilisation ein einfaches Kompartimentieren möglich.

Figur 2 zeigt einen schematischen Querschnitt durch ein Ausführungsbeispiel der Sensorvorrichtung, wobei eine Klammer **11** als Kompartimentierungsmittel **3** den Behälter **2** in einen Nebenraum **5** und einen Hauptraum **4** kompartimentiert.

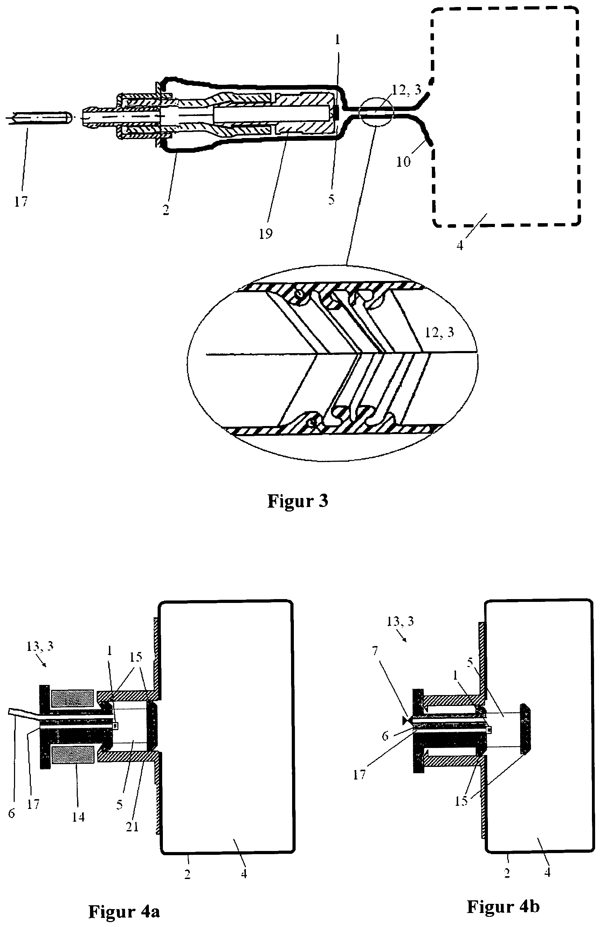

Figur 3 zeigt einen schematischen Querschnitt durch ein Ausführungsbeispiel der Sensorvorrichtung, wobei ein Druckverschluss **12**, im Vergrößerungsausschnitt im Querschnitt und geöffnet dargestellt, den Behälter in einen Nebenraum **5** und einen Hauptraum **4** kompartimentiert. Der Druckverschluss **12** kann beispielsweise in Form eines ZipLoc^{®}-Verschlusses (SC Johnson & Son, Inc., Racine, USA) ausgeführt sein.

Gemäß Figur 4a und 4b besteht eine Ausführungsform der Sensorvorrichtung aus einem Port-System **13**, welches durch Dichtungen **15** gegenüber der Atmosphäre und dem Hauptraum **4** isoliert ist. Figur 4a zeigt eine Schnittansicht durch ein Ausführungsbeispiel der Sensorvorrichtung, mit einem den optischen Sensor **1** enthaltenden Port-Systems **13** in kompartimentiertem Zustand. Durch eine Halteeinrichtung **14**, beispielsweise einen Plastikring, wird ein unbeabsichtigtes Öffnen der Kompartimentierung verhindert. Durch eine verschließbare Leitung **6** lässt sich der Nebenraum **5** evakuieren, womit eine geringere Gaskonzentration eine niedrigere Anzahl an reaktiven Teilchen bedingt, und somit eine herabgesetzte Schädigung des optischen Sensors **1.** Die verschließbare Leitung **6** ist beispielsweise thermoplastisch verschließbar. Figur 4b zeigt eine Schnittansicht durch ein Ausführungsbeispiel der Sensorvorrichtung mit Port-System **13** im geöffneten Zustand und mit Ventil **7** an der verschließbaren Leitung **6.** Durch die sich noch in der Port-Führung **21** befindliche Dichtung ist Sterilität gewährleistet.

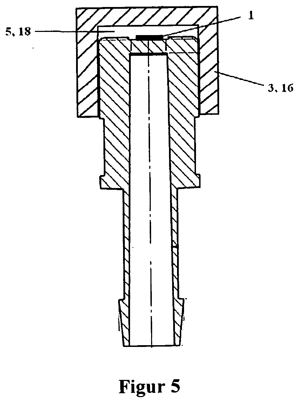

Figur 5 stellt ein weiteres Ausführungsbeispiel der Sensorvorrichtung dar. Eine Kappe **16** verschließt den Nebenraum **5** vorzugsweise gas- und fluiddicht. Der Nebenraum **5** ist mit einem wässrigen Reduktionsmittel **18** wie beispielsweise 20%ige L-Ascorbinsäurelösung befüllt. Hierdurch wird eine Radiolyse der Sensorchemie wirksam unterbunden, indem radikalische Kettenreaktionen verhindert werden. Somit kann der Sensor unter Erhalt seiner Sensitivität strahlensterilisiert werden. Weitere geeignete Reduktionsmittel sind beispielsweise die Salze der L-Ascorbinsäure, Iso-Ascorbinsäure, Verbindungen aus der Klasse der Hydrochinone oder Glutathion.

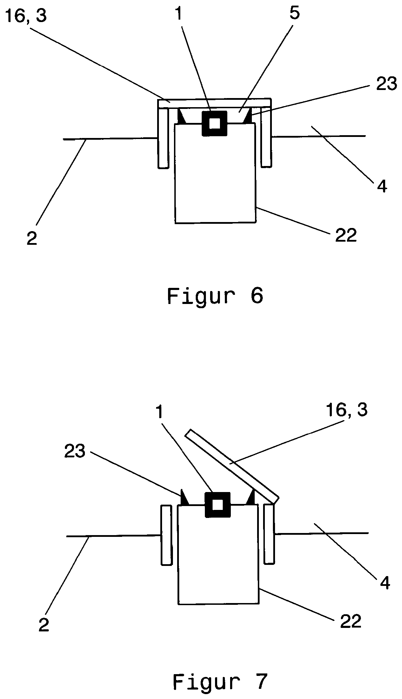

Gemäß den Figuren 6 und 7 besteht eine weitere Ausführungsform der Sensorvorrichtung aus einem Schubsystem **22**, welches eine Kappe **16** durch einen oder mehrere Vorsprünge **23** aufstößt. Figur 6 zeigt das System im kompartimentierten Zustand, Figur 7 in geöffnetem. Die Kompartimentierung kann entweder vollständig, hermetisch oder auch nur teilweise durchgeführt sein. Vorzugsweise verschließt die Kappe **16** den Nebenraum **5** vom Hauptraum **4** dicht ab, damit ein möglichst geringes Raumvolumen um den optischen Sensor **1** herum besteht. Durch entsprechende Vorrichtungen ist auch ein Vakuum im Nebenraum **5** schaffbar. Mechanische Konstruktionsmerkmale können ein Öffnen erleichtern. Das Schubsystem **22** kann beispielsweise durch eine Drehbewegung in den Hauptraum **4** schraubbar sein, somit ist ein geringerer Kraftaufwand nötig, um die Kappe **16** zu öffnen.

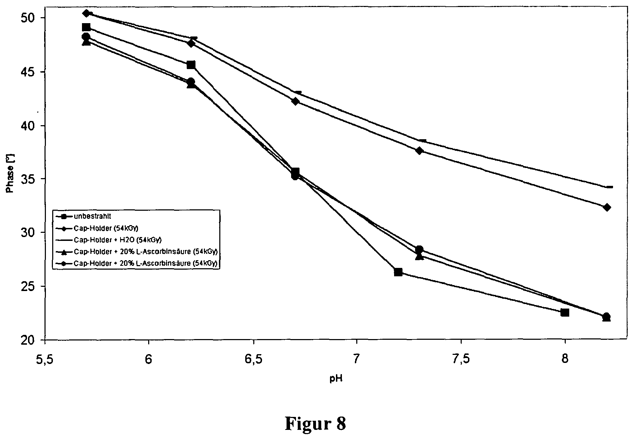

Gemäß Figur 8 besitzen erfindungsgemäße Sensorvorrichtung eine weitgehend hohe Unempfindlichkeit gegenüber Bestrahlung.

Eine Ausführungsform der vorliegenden Erfindung soll an dem nachstehenden Ausführungsbeispiel näher erläutert werden.

Beispiel:
Ein Kunststoffbehälter (Cultibag RM, Sartorius Stedim Biotech GmbH, Göttingen/Volumen: 10 L) wird mit vier pH-Sensoren (HP8, Presens GmbH, Regensburg) auf Polycarbonat-Kappen ausgestattet. Zwei der Polycarbonat-Kappen mit der Sensorchemie sind mit einer einfachen luftdicht abschließenden Schutzkappe bedeckt, bei zwei weiteren Sensorkappen wird die Schutzkappe zusätzlich noch mit zwanzigprozentiger L-Ascorbinsäure-Lsg. befüllt. Die Sensoren werden an der Wandung des Behälters angebracht, wobei die Sensorchemie dem Mittelpunkt des Behälters zugewandt ist. Anschließend wird die Vorrichtung mit 10 L Luft befüllt und dicht verschlossen. Daraufhin erfolgt eine Gamma-Bestrahlung bei 56,0 kGy (Co60-Quelle, Beta-Gamma-Service GmbH & Co. KG, Wiehl). Nach der Bestrahlung werden die Sensoren aus den Behältern entnommen und mit einem Transmitter (pH-1 mini, Presens GmbH, Regendburg) in Puffern mit pH-Werten von 5,7 bis 8,5 vermessen. Die Empfindlichkeit und damit die Güte der Sensoren entspricht der Differenz der Phasen bei pH 6,0 und pH 8,0 (siehe Figur 8).

Die Sensoren, welche mit dem Reduktionsmittel behandelt wurden, weisen eine dem unbestrahlten Sensor ähnliche Kennlinie auf. Dies entspricht einer weitgehend hohen Unempfindlichkeit gegenüber der Bestrahlung.

### Bezugszeichenliste:

- 1: optischer Sensor
- 2: Behälter
- 3: Kompartimentierungsmittel
- 4: Hauptraum
- 5: Nebenraum
- 6: verschließbare Leitung
- 7: Ventil
- 8: Schlauch
- 9: Pfropfen
- 10: Wandung
- 11: Klammer
- 12: Druckverschluss
- 13: Port-System
- 14: Halteeinrichtung
- 15: Dichtung
- 16: Kappe
- 17: Lichtwellenleiter
- 18: Reduktionsmittel
- 19: Lichtdurchlässige Kappe
- 20: Flansch
- 21: Port-Führung
- 22: Schubsystem
- 23: Vorsprung

## Patentansprüche

1. Sensorvorrichtung, umfassend mindestens einen auf porösen Matrizes basierenden optischen Sensor (1), einen vor der Benutzung mittels Strahlung zu sterilisierenden Behälter (2), wobei der Behälter (2) eine Sterilbarriere aufweist,
und ein Kompartimentierungsmittel (3), wobei das Kompartimentierungsmittel (3) derart ausgebildet ist, dass es den Behälter (2) temporär in mindestens einen Hauptraum (4) und einen den optischen Sensor (1) enthaltenden Nebenraum (5) kompartimentieren kann, und wobei das Kompartimentierungsmittel (3) derart ausgebildet ist, dass die Kompartimentierung rückgängig gemacht werden kann, ohne die Sterilbarriere des Behälters (2) zu brechen.

2. Sensorvorrichtung nach Anspruch 1, wobei der Behälter (2) durch Strahlung unter Erhalt der Funktionsfähigkeit des optischen Sensors (1) sterilisierbar ist.

3. Sensorvorrichtung nach einem der vorhergehenden Ansprüche, wobei durch das Kompartimentierungsmittel (3) der Nebenraum (5) gegenüber dem Hauptraum (4) gasdicht abgrenzbar ist.

4. Sensorvorrichtung nach einem der Ansprüche 1 bis 2, wobei das Kompartimentierungsmittel (3) derart ausgebildet ist, dass es den Nebenraum (5) gegenüber dem Hauptraum (4) partiell verschließen kann.

5. Sensorvorrichtung nach einem der vorhergehenden Ansprüche, wobei das Kompartimentierungsmittel (3) eine den optischen Sensor (1) bedeckende Kappe (16) ist.

6. Sensorvorrichtung nach einem der Ansprüche 1 bis 4 wobei das Kompartimentierungsmittel (3) ein den optischen Sensor (1) enthaltender Schlauch (8) ist.

7. Sensorvorrichtung nach einem der Ansprüche 1 bis 4, wobei der Behälter (2) eine Wandung (10) aufweist, und wobei das Kompartimentierungsmittel (3) durch die Wandung (10) des Behälters (2) ausgeführt ist.

8. Sensorvorrichtung nach Anspruch 7, wobei das Kompartimentierungsmittel (3) eine Klammer (11) ist, welche derart ausgebildet ist, dass sie die Wandung (10) zusammendrücken kann.

9. Sensorvorrichtung nach Anspruch 7, wobei das Kompartimentierungsmittel durch einen Druckverschluss (12) ausgeführt ist.

10. Sensorvorrichtung nach einem der Ansprüche 1 bis 4, wobei der Nebenraum (5) ein den optischen Sensor (1) aufnehmendes Port-System (13) ist.

11. Sensorvorrichtung nach Anspruch 10, wobei das Port-System (13) mindestens eine Dichtung (15) aufweist.

12. Sensorvorrichtung nach einem der vorhergehenden Ansprüche, wobei der Behälter (2) ein Kunststoffbehälter mit zumindest teilweiser flexibler Wandung (10) ist.

13. Sensorvorrichtung nach einem der vorhergehenden Ansprüche, wobei der Nebenraum (5) zumindest teilweise mit einem wässrigen Reduktionsmittel (18) befüllt werden kann.

## Claims

1. A sensor device, comprising at least one optical sensor (1) based on porous matrices, a container (2) to be sterilized prior to use by means of radiation, wherein the container (2) has a sterile barrier,
and a compartmentalization means (3), wherein the compartmentalization means (3) is embodied such that it can temporarily compartmentalize the container (2) in at least one main space (4) and an adjacent space (5) containing the optical sensor (1), and wherein the compartmentalization means (3) is embodied such that the compartmentalization can be undone, without breaking the sterile barrier of the container (2).

2. The sensor device according to claim 1, wherein the container (2) can be sterilized by radiation while retaining the functionality of the optical sensor (1).

3. The sensor device according to any of the foregoing claims, wherein the adjacent space (5) can be delimited in a gas-tight manner with respect to the main space (4) by the compartmentalization means (3).

4. The sensor device according to any of claims 1 to 2, wherein the compartmentalization means (3) is embodied such, that it can partially seal the adjacent space (5) with respect to the main space (4).

5. The sensor device according to any of the foregoing claims, wherein the compartmentalization means (3) is a cap (16) covering the optical sensor (1).

6. The sensor device according to any of claims 1 to 4, wherein the compartmentalization means (3) is a tube (8) containing the optical sensor (1).

7. The sensor device according to any of claims 1 to 4, wherein the container (2) has a wall (10), and wherein the compartmentalization means (3) is implemented by the wall (10) of the container (2).

8. The sensor device according to claim 7, wherein the compartmentalization means (3) is a clamp (11), which is embodied such that it can press together the wall (10).

9. The sensor device according to claim 7, wherein the compartmentalization means is implemented by a pressure closure (12) .

10. The sensor device according to any of claims 1 to 4, wherein the adjacent space (5) is a port system (13) accommodating the optical sensor (1).

11. The sensor device according to claim 10, wherein the port system (13) has at least one seal (15).

12. The sensor device according to any of the foregoing claims, wherein the container (2) is a plastic container with at least partially flexible wall (10).

13. The sensor device according to any of the foregoing claims, wherein the adjacent space (5) can be at least partially filled with an aqueous reducing agent (18).

## Revendications

1. Dispositif de détection, comprenant au moins un capteur optique (1) se basant sur des matrices poreuses, un récipient (2) à stériliser avant utilisation au moyen d'un rayonnement, dans lequel le récipient (2) présente une barrière stérile,
et un moyen de compartimentation (3), dans lequel le moyen de compartimentation (3) est réalisé de telle sorte qu'il peut compartimenter le récipient (2) temporairement en au moins un espace principal (4) et un espace secondaire (5) contenant le capteur optique (1), et dans lequel le moyen de compartimentation (3) est réalisé de telle sorte que la compartimentation peut être annulée sans briser la barrière stérile du récipient (2).

2. Dispositif de détection selon la revendication 1, dans lequel le récipient (2) peut être stérilisé par rayonnement avec conservation du bon fonctionnement du capteur optique (1).

3. Dispositif de détection selon l'une quelconque des revendications précédentes, dans lequel l'espace secondaire (5) peut être délimité de manière étanche au gaz par rapport à l'espace principal (4) par le moyen de compartimentation (3).

4. Dispositif de détection selon l'une quelconque des revendications 1 à 2, dans lequel le moyen de compartimentation (3) est réalisé de telle sorte qu'il peut fermer partiellement l'espace secondaire (5) par rapport à l'espace principal (4).

5. Dispositif de détection selon l'une quelconque des revendications précédentes, dans lequel le moyen de compartimentation (3) est un capuchon (16) recouvrant le capteur optique (1).

6. Dispositif de détection selon l'une quelconque des revendications 1 à 4, dans lequel le moyen de compartimentation (3) est un tuyau (8) contenant le capteur optique (1).

7. Dispositif de détection selon l'une quelconque des revendications 1 à 4, dans lequel le récipient (2) présente une paroi (10), et dans lequel le moyen de compartimentation (3) est conçu par la paroi (10) du récipient (2).

8. Dispositif de détection selon la revendication 7, dans lequel le moyen de compartimentation (3) est une pince (11), laquelle est réalisée de telle sorte qu'elle peut comprimer la paroi (10).

9. Dispositif de détection selon la revendication 7, dans lequel le moyen de compartimentation est conçu par une fermeture à pression (12).

10. Dispositif de détection selon l'une quelconque des revendications 1 à 4, dans lequel l'espace secondaire (5) est un système de port (13) recevant le capteur optique (1).

11. Dispositif de détection selon la revendication 10, dans lequel le système de port (13) présente au moins un joint (15).

12. Dispositif de détection selon l'une quelconque des revendications précédentes, dans lequel le récipient (2) est un récipient en plastique avec une paroi (10) au moins en partie flexible.

13. Dispositif de détection selon l'une quelconque des revendications précédentes, dans lequel l'espace secondaire (5) peut être rempli au moins en partie d'un agent de réduction aqueux (18).
